# EUROPÄISCHE PATENTSCHRIFT

(11) **EP 2 575 601 B1**
(45) Veröffentlichungstag und Bekanntmachung des Hinweises auf die Patenterteilung: **25.04.2018**
(21) Anmeldenummer: 11760392.8
(22) Anmeldetag: 30.05.2011
(51) Int. Cl.: A61B 5/01, A61B 5/02, A61B 5/053, G01G 19/414, G06F 19/00

(54) **VORRICHTUNG ZUR MODULAREN AUSWERTUNG**
DEVICE FOR MODULAR ANALYSIS
ÉQUIPEMENT D'ANALYSE MODULAIRE

(30) Priorität: 31.05.2010 DE 102010022637
(43) Veröffentlichungstag der Anmeldung: 10.04.2013
(73) Patentinhaber: seca ag, 4153 Reinach BL 1 (CH)
(72) Erfinder: KLÜCKMANN, Kristin, 23556 Lübeck (DE); HÖFLER, Martin, 22339 Hamburg (DE); MÜLLER, Manfred, 24105 Kiel (DE); BOSY-WESTPHAL, Anja, 24105 Kiel (DE)
(74) Vertreter: Klickow & Wetzel PartGmbB
(86) Internationale Anmeldenummer: PCT/DE2011/001137
(87) Internationale Veröffentlichungsnummer: WO 2011/150916

(56) Entgegenhaltungen:
- WO-A1-02/03349
- US-A- 4 053 951
- US-A1- 2003 223 905
- US-A1- 2009 005 651

## Beschreibung

Die Erfindung betrifft eine Vorrichtung mit mindestens einer Auswertungseinrichtung zur Auswertung von mindestens einem biologischen Parameter eines Lebewesens, die mindestens eine Dateneingabe zur Erfassung von Meßdaten mindestens eines Sensors aufweist, der mindestens einen biologischen Parameter des Lebewesens mißt und die mindestens eine Ausgabeeinrichtung für ein Auswertungsergebnis aufweist, wobei die Auswertungseinrichtung eine Steuereinheit und einen Programmspeicher aufweist und bei der der Programmspeicher eine Mehrzahl von Programm-Modulen bevorratet, die von der Steuereinheit in Abhängigkeit von einer extern vorgebbaren Steueranweisung wahlweise oder in teilweiser oder vollständiger Kombination miteinander derart aktivierbar sind, dass die aktivierten Programm-Module die Daten für das ausgewählte Auswertungsergebnis bereitstellen.

Derartige Vorrichtungen sind insbesondere dazu geeignet, Meßwerte hinsichtlich von Gesundheits- oder Ernährungszuständen eines Patienten bereitzustellen. Bekannte Vorrichtungen weisen hierzu einen an die jeweilige Anwendung angepassten unveränderlichen Aufbau auf und sind häufig mit einer entsprechenden allgemeinen Steuerungssoftware versehen.

Aus der US 2003/223905 A1 sowie der WO 02/03349 A1 sind jeweils Auswertungseinrichtungen zur Auswertung von mindestens einem biologischen Parameter eines Lebewesens bekannt. Es werden eine Dateneingabe sowie eine Erfassung von Messdaten mit mindestens einem Sensor unterstützt. Die Auswertungseinrichtungen weisen jeweils Steuereinheiten sowie Programmspeicher auf.

Aus der US 2009/0005651 A1 ist es bereits bekannt, auf einem einzigen Display eine Vielzahl verschiedener Messwerte zu visualisieren.

Aufgabe der vorliegenden Erfindung ist es, eine Vorrichtung der einleitend genannten Art derart zu konstruieren, dass Auswertungsergebnisse hinsichtlich vorgebbarer Frage-stellungen bereitgestellt werden.

Diese Erfindung wird erfindungsgemäß dadurch gelöst, dass der Programmspeicher eine Mehrzahl von Programmmodulen bevorratet, die von der Steuereinheit in Abhängigkeit von einer extern vorgebbaren Steueranweisung wahlweise oder in teilweiser oder vollständiger Kombination miteinander derart aktivierbar sind, dass die aktivierten Programmmodule die Daten für das ausgewählte Auswertungsergebnis bereitstellen, dass der Sensor und die Auswertungseinrichtung über eine Datenstrecke miteinander verbunden sind, dass eine Anzeigeeinrichtung und die Auswertungseinrichtung über eine Datenstrecke miteinander verbunden sind und dass die Auswertungseinrichtung eine Schnittstelle zur Eingabe von nicht messtechnisch erfassten Daten aufweist dass der Sensor und die Auswertungseinrichtung über eine Datenstrecke miteinander verbunden sind, dass eine Anzeigeeinrichtung und die Auswertungseinrichtung über eine Datenstrecke miteinander verbunden sind und dass die Auswertungseinrichtung eine Schnittstelle zur Eingabe von nicht messtechnisch erfassten Daten aufweist und dass in Abhängigkeit von den bei der Konfiguration ausgewählten Programm-Modulen ausschließlich diejenigen Messwerte bestimmt und ausgewertet werden, die für die tatsächlich ausgewählten Programm-Module benötigt werden, wobei Auswertemodule vorhanden sind, die unterschiedliche Messparameter und/oder Eingabeparameter miteinander kombinieren.
Die erfindungsgemäße Vorrichtung weist somit eine modulare Struktur derart auf, dass jeweils benötigte Hardware- oder Softwaremodule aktivierbar und miteinander verknüpfbar sind. Die Vorrichtung kann beispielsweise die Grundstruktur eines Gerätes zur Analyse der Körperzusammensetzung besitzen (Body Composition Analyzer). Weitere mögliche Anwendungen sind beispielsweise Waagen- und Längenmesseinrichtungen.
Im Gegensatz zum Stand der Technik werden durch die vorliegende Erfindung Werte für konkret vorliegende Fragestellungen beziehungsweise für die jeweiligen Untersuchungssituationen relevante Ergebnisse bereitgestellt. Es werden somit gerade nicht möglichst viele verschiedene Messwerte auf einem einzigen Display visualisiert.
Ein modularer Systemaufbau wird dadurch unterstützt, dass der Sensor und die Auswertungseinrichtung über eine Datenstrecke miteinander verbunden sind.
Ebenfalls trägt es zu einem modularen Systemaufbau bei, dass eine Anzeigeeinrichtung und die Auswertungseinrichtung über eine Datenstrecke miteinander verbunden sind.

Die Berücksichtigung von vorhandenen Daten wird dadurch unterstützt, dass die Auswertungseinrichtung eine Schnittstelle zur Eingabe von nicht-meßtechnisch erfaßten Daten aufweist.

Insbesondere ist daran gedacht, dass die Schnittstelle zur manuellen Dateneingabe ausgebildet ist.

Eine umfassende Konfigurierbarkeit wird dadurch bereitgestellt, dass der mindestens eine Sensor mindestens einen Meßparameter ausgewählt aus der Gruppe: Körpergewicht, Impedanz, Körpergröße, Blutdruck, EKG, Herzfrequenz, Blutwerte, Pulsoxymetrie, Temperatur, respiratorische Parameter, auskultatorische Parameter und / oder Energieverbrauch messtechnisch erfasst.

Eine unmittelbare Bereitstellung von Auswertungsergebnissen kann dadurch erreicht werden, dass die Auswertungseinheit mit einem Drucker gekoppelt ist.

In den Zeichnungen sind Ausführungsbeispiele der Erfindung schematisch dargestellt. Es zeigen:
- Fig. 1: ein Blockschaltbild zur Veranschaulichung des grundsätzlichen Aufbaus der Vorrichtung,
- Fig. 2: ein Anzeigebeispiel zu Auswertungsergebnissen,
- Fig. 3: ein Anzeigebeispiel zu detaillierten Auswertungsergebnissen einer ersten Detaillierungsstufe,
- Fig. 4: ein Anzeigebeispiel zu detaillierten Auswertungsergebnissen einer zweiten Detaillierungsstufe,
- Fig. 5: ein Anzeigebeispiel für Rohdaten der Auswertung,
- Fig. 6: eine detaillierte Darstellung zu Rohdaten der Auswertung,
- Fig. 7: eine perspektivische Darstellung eines Druckers zur Datenausgabe mit Adapter sowie Datenkarte und
- Fig. 8: eine weitere perspektivische Darstellung des Druckers gemäß Figur 7.

Die erfindungsgemäße Vorrichtung besitzt mindestens ein Meßgerät bzw. mindestens einen Sensor für mindestens einen der folgenden Parameter eines Patienten: Gewicht, Impedanz, Körpergröße, Blutdruck, EKG, Herzfrequenz, Blutwerte, Pulsoxymetrie, Temperatur, respiratorische Parameter, auskultatorische Parameter und / oder Energieverbrauch. Generell betreffen Meßparameter beliebige physikalische Größen des Patienten, die im Rahmen einer medizinischen Untersuchung erhoben werden. Die Meßparameter können der Auswertungseinrichtung direkt oder unter Verwendung externer Datenquellen, beispielsweise eines Laborinformationssystems, zugeführt werden.

Zusätzlich zu den Meßparametern können Eingabeparameter verwendet werden, die über eine Patientenidentifikation oder durch eine Befragung vor einer Untersuchung ermittelt werden. Dies können beispielsweise das Geschlecht und/oder das Alter und / oder die Ethnie einer Person sein.

Basierend auf den Meßparametern und/oder den Eingabeparametern werden Auswerteparameter ermittelt. Dies kann beispielsweise unter Verwendung von in einer Auswertungseinrichtung implementierter mathematischer Formeln erfolgen, die zugeordnete Werte für die Auswerteparameter ermitteln. Die Formeln können beispielsweise aus dem publizierten Stand der Technik entnommen werden oder über klinische Studien ermittelt sein. Eine Interpretation der Auswerteparameter erfolgt unter Verwendung von Referenzen. Es handelt sich bei diesen Referenzen um Normalbereiche, die in wissenschaftlichen Publikationen veröffentlicht sind oder über Reihenmessungen ermittelt werden. Ein Beispiel für eine derartige Darstellung relativ zu einem Referenzwert ist der Phasenwinkel.

Unter Verwendung von Auswertemodulen ist es möglich, unterschiedliche Auswerteparameter miteinander zu kombinieren. Die Auswertemodule dienen zur Bereitstellung der Werte für konkret vorliegende Fragestellungen. Beispielsweise kann dies der energetische Status einer Person sein.

In Abhängigkeit von einer von einem Bediener der Vorrichtung vorgenommenen Selektion erfolgt die Kombination derjenigen Auswertemodule, die für die jeweilige Untersuchungssituation relevant sind.

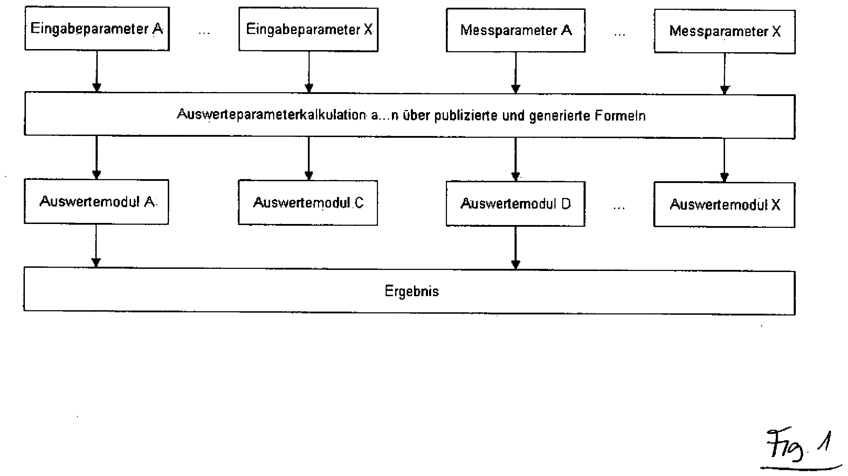

Die entsprechende Verknüpfung der Eingabeparameter und der Meßparameter über die Kalkulation der Auswerteparameter und die entsprechende Bereitstellung der Auswertemodule zur Ableitung des Ergebnisses ist in Fig. 1 veranschaulicht.

Ein Anschluß der verwendeten gerätetechnischen Komponenten kann beispielsweise über ein Funknetzwerk, insbesondere unter Verwendung von USB-Funkadaptern erfolgen. Ein Umschalten zwischen unterschiedlichen Funknetzwerken ist möglich. Ebenfalls ist ein Anschluß mehrerer Arbeitsplätze möglich, die vorzugsweise über ein Ethernet vernetzt sind.

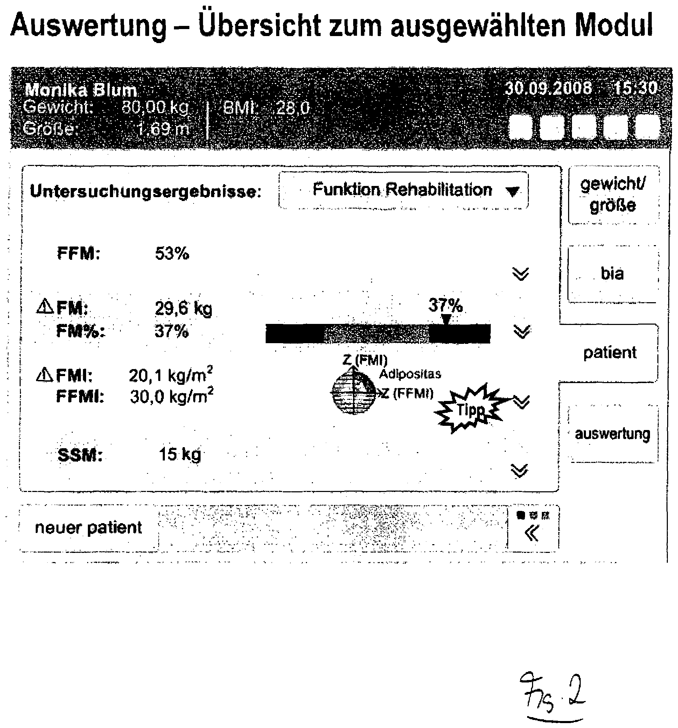

Fig. 2 veranschaulicht eine Displaydarstellung des Gerätes für eine Beispielspatientin. Hinsichtlich der Auswertung wird eine Übersicht zum ausgewählten Modul veranschaulicht.

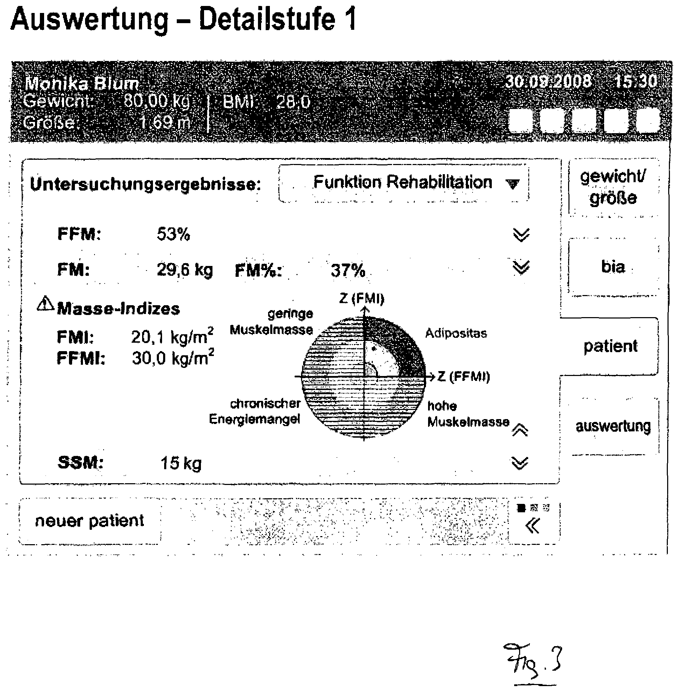

Fig. 3 veranschaulicht als Displayanzeige eine erste Detaillierungsstufe einer beispielhaften Auswertung. Über eine Visualisierung ist in einfacher Weise zu erkennen, ob ausgewählte Parameter innerhalb eines Toleranzbereiches liegen.

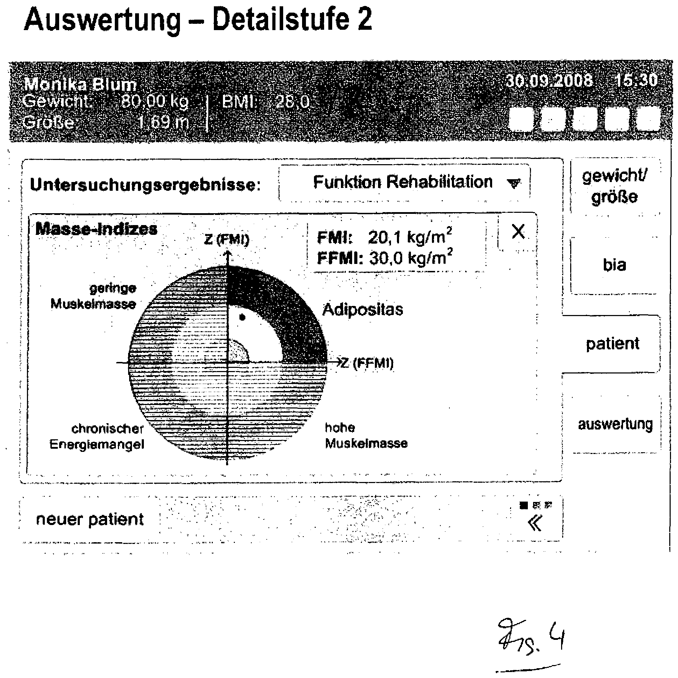

Fig. 4 veranschaulicht für die Auswertung eine zweite Detaillierungsstufe mit einer vergrößerten visualisierten grafischen Auswertung.

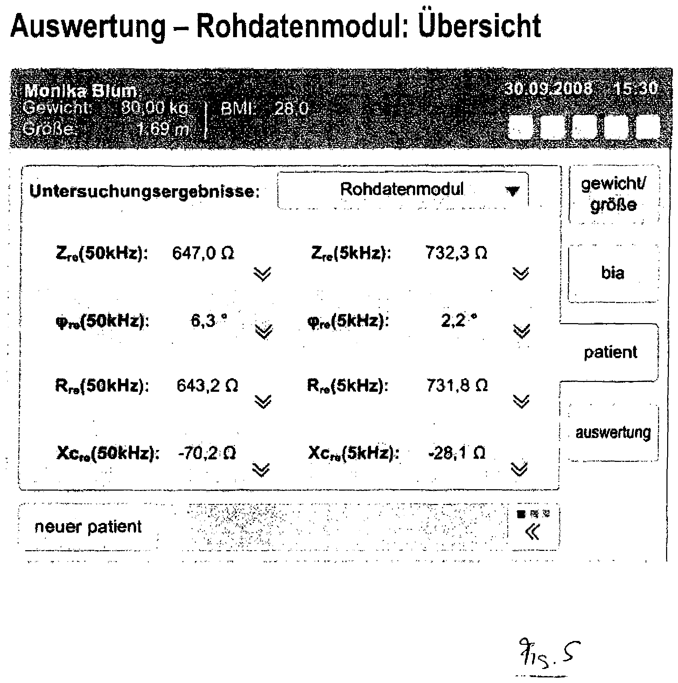

Fig. 5 zeigt eine Übersicht zu einem Rohdatenmodul der Auswertung.

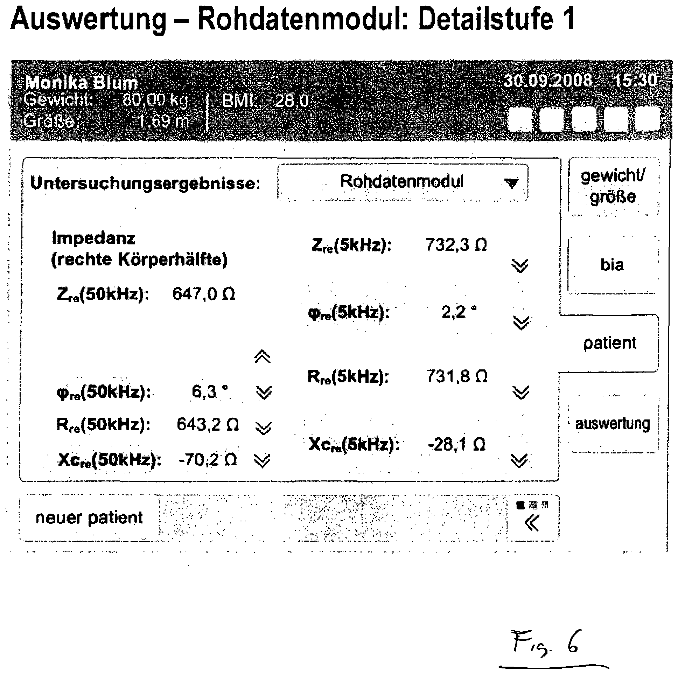

Fig. 6 zeigt zur weiteren Veranschaulichung eine Detailstufe 1 des Rohdatenmoduls der Auswertung.

Bei der erfindungsgemäßen Vorrichtung ist der jeweils verwendete Sensor bzw. das eingesetzte Meßgerät über eine Datenstrecke mit der zugeordneten Auswertungseinheit verbunden. Die Datenstrecke kann drahtlos oder leitungsgebunden realisiert werden. Sowohl die Auswertungseinheit als auch die Anzeigeeinheit werden funktionell modular strukturiert, um eine jeweils benötigte Funktionalität in einfacher Weise konfigurierbar zu machen.

Die Auswertungseinrichtung kann beispielsweise durch einen entsprechend programmierten Computer realisiert werden. Insbesondere ist auch daran gedacht, die Auswertungseinheit im Bereich eines Auswertungsteiles zu implementieren, die mit einem Drucker koppelbar ist. Hierdurch ist eine sofortige Aussage hinsichtlich der ausgewerteten Parameter möglich.

Bei der Anzeige der ermittelten Werte ist insbesondere daran gedacht, Daten, die auf bestimmte gesundheitliche Risiken hinweisen, hervorgehoben darzustellen. Dies kann beispielsweise eine vergrößerte Darstellung, eine farbliche Gestaltung oder eine zeitlich variierende Anzeige sein.

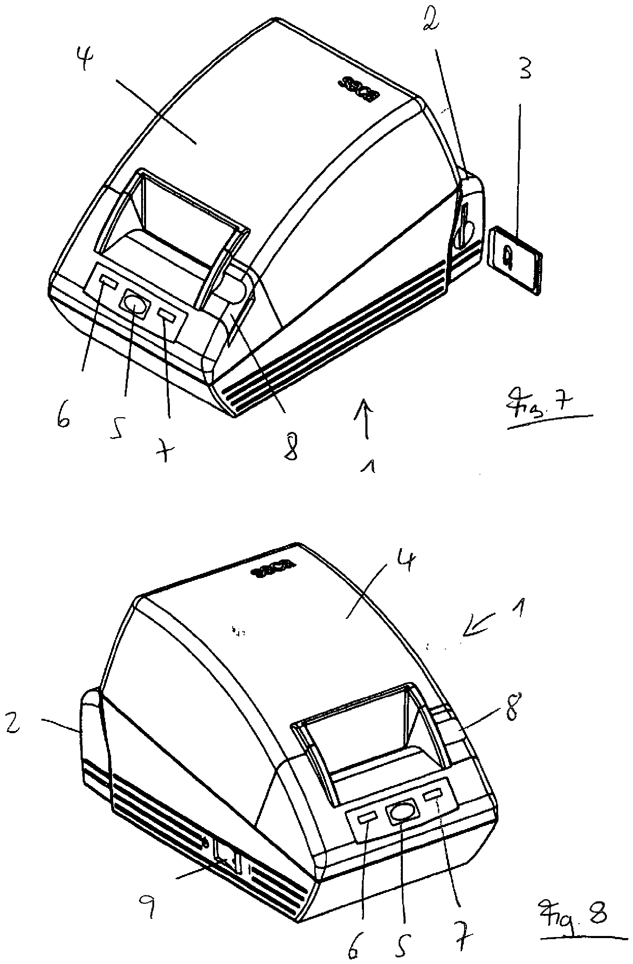

Fig. 7 zeigt einen Drucker 1, der als Teil der Auswertungseinrichtung ausgebildet ist oder die Auswertungseinheit bereitstellt oder mit der Auswertungseinrichtung koppelbar ist. Gemäß der dargestellten Ausführungsform ist der Drucker (1) mit einem Adapter (2) gekoppelt, der die Auswertungseinheit bereitstellt. Insbesondere ist auch daran gedacht, dass der Adapter (2) eine drahtlose Kommunikation mit einem oder mehreren Messgeräten ermöglicht.

Gemäß einer weiteren bevorzugten Ausführungsform ist der Adapter (2) auf eine Standard-Schnittstelle des Druckers aufgesteckt. Dies kann beispielsweise eine parallele Schnittstelle, eine serielle Schnittstelle oder ein USB-Anschluss sein.

Gemäß einer weiteren bevorzugten Ausführungsform weist der Adapter (2) eine universelle Hardwarestruktur auf und wird durch ein Zusatzelement (3) anwendungsabhängig konfiguriert. Insbesondere ist daran gedacht, dass das Zusatzelement (3) mit einer Steuerungs- und / oder Auswertungssoftware versehen ist. Beispielsweise kann das Zusatzelement (3) als eine SD-Karte ausgebildet sein.

Gemäß einer typischen Ausführungsform des Druckers (1) weist dieser eine Klappe (4) zur Ermöglichung eines Einsetzens einer Papierrolle auf. Darüber hinaus sind ein Bedienelement (5) sowie Anzeigeelemente (6, 7) vorgesehen. Zu einer Entriegelung der Klappe (4) dient eine Taste (8).

Fig. 8 zeigt den Drucker (1) in einer anderen perspektivischen Darstellung. Zur erkennen ist hier zusätzlich ein Schalter (9) für ein Ein- und Ausschalten des Gerätes.

Gemäß einer Ausführungsform der Auswertungseinrichtung ist daran gedacht, eine Selbstkonfigurierung des Gerätes in Abhängigkeit von erfassten messtechnischen Parametern vorzunehmen. Die Selbstkonfigurierung kann vollautomatisch oder als Konfigurierungsvorschlag an einen Bediener erfolgen. Im Rahmen einer Vorprüfung der Messwerte wird hierbei ermittelt, welche Module in Abhängigkeit von der messtechnisch erfassten Situation für den konkreten Anwendungsfall zweckmäßig sind.

Gemäß einer anderen Ausführungsvariante ist daran gedacht, zur Minimierung der erforderlichen Auswertungszeit in Abhängigkeit von den bei der Konfiguration ausgewählten Modulen ausschließlich diejenigen Messwerte zu bestimmen und auszuwerten, die für die tatsächlich aktivierten Module benötigt werden.

Gemäß einem Ausführungsbeispiel kann der Drucker (1) unter Verwendung des Adapters (2) automatisch diejenigen Parameter eines Programmmoduls berechnen, für die benötigte Eingabewerte und Messwerte vorliegen. Ebenfalls ist daran gedacht, dass über den Adapter (2) die auf einem Ausdruck des Druckers (1) dargestellten Module durch eine vorab im Gerät konfigurierte Modulauswahl festgelegt werden.

Ein Programmmodul wird typischerweise durch eine vorbestimmte Anzahl an Parametern definiert. Ein Parameter ist hierbei ein Messwert oder ein kalkulierter Wert, der eine Aussage über den Gesundheitszustand einer Person liefert. Verschiedene Parameter können über eine Auswahl frei zu einem benutzerspezifischen Modul zusammengefügt werden.

## Patentansprüche

1. Vorrichtung mit mindestens einer Auswertungseinrichtung (2) zur Auswertung von mindestens einem biologischen Parameter eines Lebewesens, die mindestens eine Dateneingabe zur Erfassung von Messdaten mindestens eines Sensors aufweist, der mindestens einen biologischen Parameter des Lebewesens misst, und die mindestens eine Ausgabeeinrichtung (1) und eine Anzeigeeinrichtung (6,7) für ein Auswertungsergebnis aufweist, wobei die Auswertungseinrichtung eine Steuereinheit und einen Programmspeicher aufweist, wobei der Programmspeicher eine Mehrzahl von Programm-Modulen bevorratet, die von der Steuereinheit in Abhängigkeit von einer extern vorgebbaren Steueranweisung wahlweise oder in teilweiser oder vollständiger Kombination miteinander derart aktivierbar sind, dass die aktivierten Programm-Module die Daten für das ausgewählte Auswertungsergebnis bereitstellen, wobei der Sensor und die Auswertungseinrichtung über eine Datenstrecke miteinander verbunden sind, und die Anzeigeeinrichtung und die Auswertungseinrichtung über eine Datenstrecke miteinander verbunden sind und wobei die Auswertungseinrichtung eine Schnittstelle zur Eingabe von nicht messtechnisch erfassten Daten aufweist und die Auswertungseinrichtung eingerichtet ist, in Abhängigkeit von den bei der Konfiguration ausgewählten Programm-Modulen ausschließlich diejenigen Messwerte zu bestimmen und auszuwerten, die für die tatsächlich ausgewählten Programm-Module benötigt werden, **dadurch gekennzeichnet, dass** Auswertemodule vorhanden sind, die eingerichtet sind, unterschiedliche Messparameter und/oder Eingabeparameter miteinander zu kombinieren.

2. Vorrichtung nach Anspruch 1, **dadurch gekennzeichnet, dass** die Schnittstelle zur manuellen Dateneingabe ausgebildet ist.

3. Vorrichtung nach einem der Ansprüche 1 oder, **dadurch gekennzeichnet, dass** der mindestens eine Sensor mindestens einen Messparameter ausgewählt aus der Gruppe: Körpergewicht, Impedanz, Körpergröße, Blutdruck, EKG, Herzfrequenz, Blutzusammensetzung, Pulsoxymetrie, Temperatur, respiratorische Parameter, auskultatorische Parameter und/oder Energieverbrauch messtechnisch erfasst.

4. Vorrichtung nach einem der Ansprüche 1 bis 3, **dadurch gekennzeichnet, dass** die Auswertungseinheit mit einem Drucker (1) gekoppelt ist.

5. Vorrichtung nach Anspruch 4, **dadurch gekennzeichnet, dass** die Auswertungseinheit im Bereich eines Adapters (2) des Druckers (1) lokalisiert ist.

6. Vorrichtung nach Anspruch 4 oder 5, **dadurch gekennzeichnet, dass** der Adapter (2) über ein Zusatzelement (3) konfigurierbar ist.

7. Vorrichtung nach einem der Ansprüche 1 bis 6, **dadurch gekennzeichnet, dass** die Auswertungseinrichtung zur Aktivierung und Deaktivierung von Sensoren und / oder Auswertungsmodulen für die Sensoren ausgebildet ist.

## Claims

1. Apparatus having at least one evaluation device (2) for evaluating at least one biological parameter of a living being, said evaluation device having at least one data input for capturing measurement data from at least one sensor, said sensor measuring at least one biological parameter of the living being, and said evaluation device having at least one output device (1) and an indication device (6, 7) for an evaluation result, wherein the evaluation device has a control unit and a program memory, wherein the program memory stores a multiplicity of program modules which are activatable by the control unit, selectively or in partial or complete combination with one another, depending on an externally predeterminable control instruction in such a way that the activated program modules provide the data for the selected evaluation result, wherein the sensor and the evaluation device are connected to one another by way of a data line and the indication device and the evaluation device are connected to one another via a data line and wherein the evaluation device has an interface for entering data that is not captured by measurement and the evaluation device is configured to determine and evaluate, depending on the program modules selected during the configuration, only those measurement values that are required for the actually selected program modules, **characterized in that** evaluation modules are present, said evaluation modules being configured to combine different measurement parameters and/or input parameters with one another.

2. Apparatus according to Claim 1, **characterized in that** the interface is embodied for manual data entry.

3. Apparatus according to either of Claims 1 or, **characterized in that** the at least one sensor captures by measurement at least one measurement parameter selected from the group of: bodyweight, impedance, body height, blood pressure, ECG, heart rate, blood composition, pulse oximetry, temperature, respiratory parameters, auscultatory parameters and/or energy consumption.

4. Apparatus according to any one of Claims 1 to 3, **characterized in that** the evaluation unit is coupled to a printer (1).

5. Apparatus according to Claim 4, **characterized in that** the evaluation unit is localized in the region of an adapter (2) of the printer (1).

6. Apparatus according to Claim 4 or 5, **characterized in that** the adapter (2) is configurable by way of a supplementary element (3).

7. Apparatus according to any one of Claims 1 to 6, **characterized in that** the evaluation device is embodied to activate and deactivate sensors and/or evaluation modules for the sensors.

## Revendications

1. Dispositif, doté d'au moins un système d'analyse (2) destiné à analyser au moins un paramètre biologique d'un être vivant, qui comporte au moins une saisie de données destinée à enregistrer des données mesurées d'au moins un capteur, lequel mesure au moins un paramètre biologique de l'être vivant, et qui comporte au moins un système d'édition (1) et un système d'affichage (6, 7) pour un résultat d'analyse, le système d'analyse comportant une unité de commande et une mémoire de programmes, la mémoire de programme stockant une pluralité de modules de programmes, lesquels sont activables par l'unité de commande en fonction d'une instruction de commande prédéfinissable en externe, sélectivement ou en association partielle ou complète les uns avec les autres, de telle sorte que les modules de programmes activés mettent à disposition les données pour le résultat d'analyse sélectionné, le capteur et le système d'évaluation étant connectés l'un à l'autre par l'intermédiaire d'un trajet de données et le système d'affichage et le système d'analyse étant connectés l'un à l'autre par l'intermédiaire d'un trajet de données et le système d'évaluation comportant une interface destinée à saisir des données non enregistrées par technique de mesure et le système d'évaluation étant aménagé pour déterminer et pour analyser, en fonction des modules de programmes sélectionnés lors de la configuration, exclusivement les valeurs mesurées qui sont nécessaires pour les modules de programmes effectivement sélectionnés, **caractérisé en ce que** des modules de programme sont présents, lesquels sont aménagés pour associer différents paramètres de mesure et/ou paramètres de saisie.

2. Dispositif selon la revendication 1, **caractérisé en ce que** l'interface est conçue pour la saisie manuelle de données.

3. Dispositif selon l'une quelconque des revendications 1 ou, **caractérisé en ce que** l'au moins un capteur enregistre par technique de mesure au moins un paramètre de mesure, sélectionné dans le groupe comprenant : le poids corporel, l'impédance, la taille corporelle, la pression artérielle, l'ECG, la fréquence cardiaque, la composition sanguine, l'oxymétrie par détection de pulsations, la température, des paramètres respiratoires, des paramètres auscultatoires et/ou une consommation énergétique.

4. Dispositif selon l'une quelconque des revendications 1 à 3, **caractérisé en ce que** l'unité d'analyse est couplée à une imprimante (1) .

5. Dispositif selon la revendication 4, **caractérisé en ce que** l'unité d'analyse est localisée dans la région d'un adaptateur (2) de l'imprimante (1).

6. Dispositif selon la revendication 4 ou 5, **caractérisé en ce que** l'adaptateur (2) est configurable par l'intermédiaire d'un élément additionnel (3).

7. Dispositif selon l'une quelconque des revendications 1 à 6, **caractérisé en ce que** l'unité d'analyse est conçue pour l'activation et la désactivation de capteurs et/ou de modules d'analyse pour les capteurs.
